# EUROPÄISCHE PATENTANMELDUNG

(11) **EP 3 632 896 A1**
(43) Veröffentlichungstag der Anmeldung: **08.04.2020**
(21) Anmeldenummer: 18197966.7
(22) Anmeldetag: 01.10.2018
(51) Int. Cl.: C07C 319/20, C07C 231/06, B01J 31/02, C07C 323/58, C07C 237/06

(54) **HERSTELLUNG VON AMINOSÄUREN AUS IHREN AMINONITRILEN**

(71) Anmelder: Evonik Operations GmbH, 45128 Essen (DE)
(72) Erfinder: GEIST, Lucas, 63579 Freigericht (DE); HASSEBERG, Hans-Albrecht, 63584 Gründau-Lieblos (DE); REUS, Christian, 63579 Freigericht (DE)
(74) Vertreter: Evonik Patent Association

(57) **Zusammenfassung**

Die Erfindung betrifft ein Verfahren zur Herstellung von α-Aminosäureamid, insbesondere von Methioninamid, durch Hydrolyse des entsprechenden α-Aminosäurenitrils, insbesondere von Methioninnitril, in Gegenwart eines Ketons und von Guanidin als Katalysator, sowie ein Verfahren zur Herstellung der entsprechenden α-Aminosäure, insbesondere von Methionin, unter Einbeziehung dieses Verfahrens.

## Beschreibung

Aminosäuren haben insbesondere aufgrund ihrer Funktion als Eiweißbausteine für die Ernährung von Tier und Mensch eine fundamentale Bedeutung Futtermittel in der Nutztierhaltung werden daher zusätzlich mit Aminosäuren, z. B. DL-Methionin und L-Lysin, angereichert, wodurch deren Nährwert erhöht wird.

DL-Methionin ist eine essenzielle Aminosäure, die mit der Nahrung aufgenommen werden muss. Als Futtermittelzusatz trägt sie zu einer effizienten, gesunden und umweltschonenden Ernährung von landwirtschaftlichen Nutztieren, insbesondere von Geflügel und Schweinen, bei. Sie ist damit auch ein wichtiger Baustein, wenn es um die nachhaltige Versorgung einer wachsenden Weltbevölkerung mit tierischem Protein geht. Somit kommt einer kostengünstigen und auch großindustriell gut durch führbaren Synthesemethode für DL-Methionin eine hohe Bedeutung zu.

### Stand der Technik:

Im industriellen Maßstab wird Methionin chemisch über die Bucherer-Bergs-Reaktion hergestellt, die eine Variante der Strecker-Synthese darstellt. Dabei werden die Ausgangssubstanzen 3-Methylmercaptopropanal (hergestellt aus 2-Propenal und Methylmercaptan), Blausäure (Cyanwasserstoff), Ammoniak und Kohlendioxid zum 5-(2-Methylmercaptoethyl)-hydantoin (Methioninhydantoin) umgesetzt. Die Hydrolyse des Hydantoins erfordert harsche Bedingungen und stöchiometrische Mengen an einer Base, meist Natriumhydroxid oder Kaliumhydroxid bzw. Kaliumcarbonat. Nach einem altbekannten Verfahren wird Methionin im Hydrolysat durch Neutralisation mit Schwefelsäure aus seinem Natriumsalz freigesetzt, welches als Präzipitat aus der Natriumsulfat enthaltenden Mutterlauge abfiltriert werden kann. Das Koppelprodukt Natriumsulfat muss anderweitig verwertet oder entsorgt werden.
Nach dem bekannten Degussa-Kaliumcarbonatkreislaufverfahren wird Methionin schließlich durch Behandlung des Hydrolysats mit Kohlendioxid aus seinem Kaliumsalz freigesetzt, wobei das Methionin-Präzipitat aus der Kaliumcarbonat und Kaliumhydrogencarbonat enthaltenden Mutterlauge abfiltriert werden kann (US 5,770,769). Letzteres kann zwar zurückgewonnen werden, doch ist dafür ein Kreislauf einer großen Menge salzhaltiger Lösung erforderlich.
Bedingt durch den mit der Zeit ansteigenden Nebenproduktpegel in der zu recyclierenden wässrigen Mutterlauge ist es notwendig nicht unerhebliche Mengen der Mutterlauge aus dem Verfahren auszuschleusen, was den zusätzlichen Aufwand einer Aufarbeitung oder Entsorgung mit sich bringt.
Zum anderen sind die Bedingungen der Hydantoinbildung und der Hydantoinhydrolyse mit Temperaturen von bis zu über 200 °C harsch und energieintensiv, so dass weiterhin Bedarf an einer großindustriell durchführbaren Methode bestand, die die genannten Nachteile nicht oder kaum mehr aufweist.

Eine bekannte Alternative zur Bucherer-Bergs-Reaktion stellt der Herstellungsweg via Methioninnitril, dem Aminonitril von 3-Methylmercaptopropionaldehyd (MMP-AN) und dessen anschließende Verseifung zu Methionin mit Hilfe stöchiometrischer Mengen Kalilauge oder Schwefelsäure nach Strecker dar. Aber auch diese Route macht der hohe stöchiometrische Salzabfall, der durch die ebenfalls nötige Neutralisation bedingt ist, unattraktiv für ein großindustrielles Herstellungsverfahren.
Alternativ können die Aminonitrile in Gegenwart eines Ketons wie z.B. Aceton und einer starken Hydroxidbase wie z.B. NaOH als Katalysator mit hoher Selektivität schon bei Raumtemperatur in die entsprechenden alpha-Aminoamide umgewandelt werden (z.B. Bull. Soc. Chim. Fr. 1978, 3-4, II-177 bzw. DE2637204). Eine anschließende Verseifung des Methioninamids mittels Alkalien liefert unter vergleichsweise milden Bedingungen von z:B. 100 - 120°C das Alkalimetallsalz des Methionins (z.B. gemäß WO 94/08957 A1, Beispiel 18), aus dem wiederum durch Neutralisation mit Säure Methionin erst freigesetzt werden muss unter Bildung entsprechender Salze als unerwünschtem Koppelprodukt (Schema 1). Die Ausbeuten an Methioninamid bzw. Methionin liegen bei ca. 95- 97 % und sind damit zwar hoch jedoch nicht quantitativ.
Aber auch wenn die anschließende Verseifung des Amides direkt zur Carbonsäure über ein Neutralverseifungsverfahren mit einem heterogenen Katalysator (z.B. TiO₂) angewendet wird (Schema 1, z.B. DE 60127538 T2), so ist diese Route dennoch nicht salzfrei, da 10-20 mol% einer starken Base wie z.B. NaOH in der Amidbildungsreaktion eingesetzt werden müssen. Diese Base muss durch physikalisch-chemische Methoden wie z.B. lonenaustauscherharze wieder abgetrennt werden.

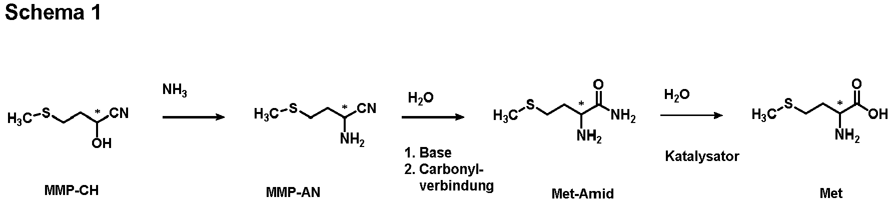

Alternativ ist die Verwendung von polymer gebundenen Hydroxidbasen bekannt (z.B. FR2785609). Diese haben jedoch den Nachteil, dass sie häufig regeneriert werden müssen, was wiederum zu Salzabfall führt.
Darüberhinaus sind im Stand der Technik mehrere Verfahrensvarianten für andere Aminosäureamide außer Methioninamid offenbart, die sich auch die carbonylkatalysierte alkalische Hydrolyse der betreffenden Aminonitrile zu Nutze machen.

JP2001-199947A offenbart ein Verfahren zur Hydrolyse von Aminosäurenitril zu Aminosäureamiden bzw. Aminosäuren. Dabei wird zunächst aus einem Cyanhydrin mit Ammoniak ein entsprechendes Aminonitril hergestellt. Dabei werden Alkalimetallhydroxide wie Natriumhydroxid oder Kaliumhydroxid in einem Vorzugsbereich von 0,01 bis 0,10 Molequivalenten (Moleq) pro ein Moleq Cyanhydrin und Methylethylketon oder Aceton als Carbonylkatalysator verwendet und in einem Temperaturbereich von 0 bis 20 °C zur Reaktion gebracht. Alaninamid, Valinamid und Phenylglycinamid wurden auf diese Weise erhalten. Die Reaktion von Methioninnitril wurde nicht explizit untersucht.

JP 2001- 247529 A offenbart ebenfalls ein Verfahren zur Hydrolyse von Aminosäurenitril zu Aminosäureamiden. Im Beispiel beschrieben ist nur ein Verfahren zur Herstelllung von tert.Leucinamid. Dabei werden Alkalimetallhydroxide wie Natriumhydroxid oder Kaliumhydroxid vorzugsweise in einem überaus breiten Vorzugsbereich von 0,01-10 Moleq (0,5 Moleq im Beispiel) pro ein Moleq Aminonitril und niedermolekulare Ketone, insbesondere Aceton als Carbonylkatalysator verwendet, bei Temperaturen von ca. 20 - 30°C und teils sehr langen Reaktionszeiten von 1-100 Stunden. Im Falle von tert-Leucinamid wurden auf diese Weise jedoch nur 83 bis 91% Ausbeute erhalten. Ein Verfahren zur Herstellung von Methioninamid ist nicht offenbart. Der relativ hohe Basenanteil von z.B. 0,5 Moleq NaOH erfordert wiederum einen relativ hohen Säureanteil, der zur Neutralisation und Isolierung von neutralem Methionin gebraucht wird. Das führt zu erheblichem Anfall von Salz als Beiprodukt, was unter ökonomischen wie ökologischen Gesichtspunkten nachteilig ist.

JP5613162B A offenbart schließlich ein Verfahren zur Herstellung des Salzes von 2-Aminobuttersäureamid mit einer anorganischen Säure durch Hydrolyse von 2-Aminobuttersäurenitril mittels einer starken anorganischen Base und einem Keton (Aceton oder Methylethylketon) als Lösungsmittel und damit in großer Menge. Dabei werden Alkalimetallhydroxide wie Natriumhydroxid oder Kaliumhydroxid in einem Vorzugsbereich von 0,005-0,10 Mol pro ein Mol Cyanhydrin und Methylethylketon oder Aceton als Carbonylkatalysator verwendet und bei einer Temperatur von 20 °C 7 Stunden lang (Beispiele) zur Reaktion gebracht. Am Ende wird durch Zugabe einer starken Mineralsäurelösung das Mineralsäuresalz des 2-Aminobuttersäureamid erzeugt. Auch diese Variante ist aufgrund des hohen Anfalls von Salz als Beiprodukt nachteilig.

CN102070473 A offenbart schließlich ein Verfahren zur Herstellung von 2-Amino-3-methylbuttersäureamid (Valinamid) durch Hydrolyse von 2- Amino-3-methyl-buttersäurenitril (Valinnitril) mittels einer starken anorganischen Base und einem Keton (Aceton oder Methylethylketon) als Carbonylkatalysator. Dabei werden Natriumhydroxid, Kaliumhydroxid oder Bariumhydroxid im Gewichtsverhältnis von 0,01-1,0 :1 und bevorzugt 0,05-0,50 :1 bezogen auf die Menge an Keton und das Keton im Verhältnis von 0,5-2,0 Moleq pro 1 Moleq Valinnitril und bei einer Temperatur von -10 bis 10 °C 5-8 Stunden lang (Beispiele) zur Reaktion gebracht. Am Ende wird durch Extraktion mit organischen Lösungsmittel (Chloroform) das Valinamid aus der wässrigen Salzlösung entfernt, was mit verringerten Ausbeuten von nur ca. 63-85 % einhergeht. Auch diese Variante ist aufgrund des hohen Anfalls von Salz als Beiprodukt und der Verwendung von organischem Lösungsmittel nachteilig.

### Aufgabe:

Die der vorliegenden Erfindung grundsätzlich zugrundeliegende Aufgabe war demzufolge die Bereitstellung eines möglichst einfachen chemischen Verfahrens zur Herstellung von D,L-Aminosäuren, insbesondere von D,L-Methionin, bei dem weniger harsche Bedingungen als in der klassischen Methode über das Hydantoin ermöglicht werden. Dabei sollte gleichzeitig der Zwangsanfall von Salzen als Koppelprodukt der klassischen Verfahren möglichst vermieden werden. Eine Teilaufgabe in diesem Zusammenhang war es, ein Verfahren zur möglichst salzarmen Herstellung von D,L-Aminosäureamiden, insbesondere von D,L- Methioninamid bereitzustellen, welches dann durch Kopplung mit einem Verfahren zur Neutralverseifung von D,L-Aminosäureamiden zu einem möglichst salzarmen Gesamtverfahren zur Herstellung von D,L-Aminosäuren, insbesondere von D,L- Methionin ergänzbar ist.

### Lösung:

Diese grundlegende Teilaufgabe wurde gelöst durch das Bereitstellen eines Verfahrens zur Herstellung von α-Aminosäureamid, insbesondere von Methioninamid, durch Hydrolyse von α-Aminosäurenitril, insbesondere von Methioninnitril, in Gegenwart eines Ketons und von Guanidin als Katalysator.
Überraschend war insbesondere, dass bereits mit sehr kleinen Anteilen von beispielsweise 0,01 bis 0,1 Moleq der starken organischen Stickstoffbase Guanidin unter Carbonylkatalyse bei moderaten Bedingungen Methioninnitril in hoher bis quantitativer Ausbeute zu Methioninamid hydrolysiert werden kann (vgl. Ergebnisse in Tabelle 1; mit Guanidin ist in diesem Zusammenhang die Guanidinbase, in den Beispielen freigesetzt aus Guanidiniumchlorid, gemeint)). So wurden bereits mit nur 0,1 Moleq Guanidin bei 1,2 Moleq Aceton 100 % Umsatz bei 99 % Selektivität erzielt, was einer praktisch quantitativen Ausbeute entspricht. Derart kleine Mengen Guanidin können auch während der nachfolgenden Hydrolysestufe vom Methioninamid zum Methionin in vorteilhafter Weise in der Reaktionsmischung zur Unterstützung der Hydrolysereaktion belassen werden und danach unter Ausnutzung der grundverschiedenen Löslichkeiten vom schwerlöslichen Methionin abgetrennt werden, um hernach in die Hydrolysestufe zum Methioninamid zurückgeführt zu werden. Alternativ ist auch eine Abtrennung aus dem erzeugten Methioninamid möglich.

Durch Weiterhydrolyse der nach dem erfindungsgemäßen Verfahren insbesondere mit sehr kleinen Anteilen von Guanidin erhaltenen Aminosäureamide, insbesondere von Methioninamid, mit Hilfe eines neutralen, z.B.TiO₂-haltigen, Katalysators wird eine salzarme Route zu Aminosäuren, insbesondere zu Methionin, aus den entsprechenden Aldehyden ermöglicht (vgl. Beispiel 4).

Die genannte Aufgabe wurde insbesondere gelöst durch Bereitstellen eines Verfahrens zur Herstellung von Methioninamid gekennzeichnet durch folgende Schritte:
a. Herstellung von Methioninnitril durch Umsetzung von Methylmercaptopropionaldehyd (MMP) mit Blausäure und Ammoniak und/oder durch Umsetzung des entsprechenden MMP-Cyanhydrins (MMP-CH) mit Ammoniak zu einem Methioninnitril enthaltendem Reaktionsgemisch,
b. optional wenigstens teilweise Abtrennung von restlichem Ammoniak aus dem Reaktionsgemisch aus a.,
c. Hydrolyse des Methioninnitrils zum Methioninamid im Reaktionsgemisch aus a. oder b. in Gegenwart eines Ketons und von Guanidin als Katalysator und
d. optional Abtrennung des Ketons und/oder des Guanidins aus dem Reaktionsgemisch aus c.

Dabei werden die in Schritt a. verwendeten Reaktanden MMP und HCN vorzugsweise äquimolar, jedoch mit bis zu 1,03 Moleq HCN bezogen auf 1 Moleq MMP eingesetzt. Ammoniak wird in Schritt a. dabei bevorzugt in Mengen von 1 bis 10 Moleq Ammoniak pro 1 Moleq der molaren Summe aus Methylmercaptopropionaldehyd und MMP-Cyanhydrin eingesetzt. Wird also ausschließlich von vorgebildetem MMP-CH ausgegangen bezieht sich die Ammoniakmenge ausschließlich auf MMP-CH. Wird im anderen Extremfall ausschließlich von MMP und HCN ausgegangen, so bezieht sich die Ammoniakmenge ausschließlich auf MMP.
Ein solches Verfahren bietet den großen Vorteil, dass man ausgehend von den üblichen Rohstoffen der Methioninsynthese, MMP, HCN und Ammoniak unter Verwendung der einfachen Stickstoffbase Guanidin , die obendrein recyclierbar ist, zu Methioninamid gelangt, der direkten Vorstufe des Futtermitteleinsatzstoffes Methionin.

Die oben genannte grundsätzliche Aufgabe wurde des Weiteren gelöst durch Bereitstellen eines Verfahrens zur Herstellung des eigentlichen kommerziell verwertbaren Endproduktes Methionin gekennzeichnet durch folgende Schritte:
a. Herstellung von Methioninnitril durch Umsetzung von Methylmercaptopropionaldehyd (MMP) mit Blausäure und Ammoniak und/oder durch Umsetzung des entsprechenden MMP-Cyanhydrins (MMP-CH) mit Ammoniak zu einem Methioninnitril enthaltenden Reaktionsgemisch,
b. optional wenigstens teilweise Abtrennung von restlichem Ammoniak aus dem Reaktionsgemisch aus a.,
c. Hydrolyse von Methioninnitril zum Methioninamid im Reaktionsgemisch aus a. oder b. in Gegenwart eines Ketons und von Guanidin als Katalysator,
d. optional wenigstens teilweise Abtrennung des Ketons Guanidins aus dem Reaktionsgemisch aus c. und
e. anschließender Hydrolyse des Methioninamids aus c. oder d. in Gegenwart eines basischen oder neutralen Katalysators,
f. Einengen des aus e. erhaltenen Hydrolysats zur Aufkonzentrierung und zur Entfernung von Ammoniak und restlichem Keton,
g. Neutralisation des aus f. erhaltenen aufkonzentrierten Hydrolysats bis zu einem pH-Wert von 7 bis 9 (gemessen mit einem pH-Meter mit Einstabmesskette) durch Zugabe entsprechender Mengen an Säure, vorzugsweise CO₂, zur Ausfälllung von Methionin und gleichzeitiger Erzeugung einer Mutterlauge, die das zur zugegebenen Säure korrespondierende Guanidiniumsalz, vorzugsweise Guanidiniumcarbonat, enthält, und
h. Abtrennung des Methionins aus der Mutterlauge.

Dabei können in Schritt g. die Bedingungen der Fällung von Methionin aus einem Kaliummethioninat enthaltenden Hydrolysat aus dem Hydantoinverfahren gemäß EP780370 A2 analog verwendet werden.
Das Verfahren kann sowohl im Batch als auch vorzugsweise kontinuierlich betrieben werden. Bei der kontinuierlichen Fahrweise können die Schritte a. bis f. bis zum Guanidinium-methioninat in Anlehnung an das in der WO 94/08957 A1 und dort insbesondere in Beispiel 22 und der Figur dargestellte Verfahren zur Herstellung von Natriummethioninat durchgeführt werden. Dabei können die Schritte e. bis f. gemeinsam in einer kontinuierlich betriebenen Verseifungskolonne durchgeführt werden, bei dem die, im vorliegenden Fall Guanidin enthaltende, Methioninamidlösung von oben in die Verseifungskolonne eingetragen wird, während die Guanidinmethioninat und Methionin enthaltende Verseifungsbrühe im Sumpf abgeführt wird zur Weiterbearbeitung in Schritt g. bis h..

In diesem Zusammenhang bevorzugt werden Verfahren der vorstehend bezeichneten Art, dadurch gekennzeichnet, dass bei der Hydrolyse des Methioninnitrils 0,01 bis 1,2, vorzugsweise 0,05 bis 1,0, besonders bevorzugt 0,08 bis 0,5, noch mehr bevorzugt von 0,08 bis 0,12 Moleq Guanidin eingesetzt werden. Dabei gewährleisten insbesondere sehr kleine Mengen von 0,08 bis 0,12 Moleq Guanidin, dass entsprechend wenig an Guanidiniumsalzen als Koppelprodukt anfallen können, was von großem Vorteil ist.

Ebenfalls bevorzugt werden Verfahren der vorstehend bezeichneten Art, dadurch gekennzeichnet, dass bei der Hydrolyse des Methioninnitrils, im Speziellen gemäß Schritt c., als Keton Aceton, Methyl-ethylketon, Diethylketon, Methyl-propylketon, Methyl-isopropylketon, Ethyl-propylketon, Cyclopentanon, Cyclohexanon oder 4-Piperidon, vorzugsweise Aceton, Methyl-ethylketon oder 4-Piperidon eingesetzt werden. Alle diese Carbonylverbindungen sind kommerziell problemlos erhältlich. Der Preis ist dabei sekundär, weil die Carbonylverbindungen als Katalysatoren eingesetzt werden, d.h. bis auf kleinere Verluste nicht verbraucht sondern zurückgewonnen und wiederverwendet werden.
Die Ketone bilden mit vorhandenem Methioninamid in Anteilen von ca.1-3 Mol % Imidazolidinone, im Falle von Aceton das 2,2-Dimethyl- 5-(2'-methylthio) ethylimidazolidin-4-on (IM2):

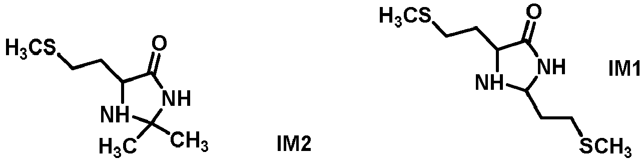

Das analog in noch geringeren Anteilen aus kleinen vorhandenen Anteilen von MMP mit Methioninamid gebildete Imidazolidinon IM1 war hier nicht nachweisbar. Diese Nebenprodukte werden aber in einem Gesamtverfahren der erfindungsgemäßen Methioninherstellung bei der anschließenden Stufe der Hydrolyse des Methioninamids ebenfalls problemlos zu Methionin hydrolysiert, so dass dadurch keine Ausbeuteverluste entstehen.

Dabei ebenfalls besonders bevorzugt werden Verfahren der vorstehend bezeichneten Art, dadurch gekennzeichnet, dass die Hydrolyse des Methioninnitrils, im Speziellen in Schritt c., bezogen auf das Nitril in Gegenwart von 15 bis 50 Moleq H₂O, 0,1 bis 1,5 Moleq Keton und 0 bis 9 Moleq NH₃, sowie bei einer Temperatur zwischen 10 und 60°C und in einer Reaktionszeit zwischen 10 und 180 Minuten durchgeführt wird.

Wie der Vergleich der Hydrolyseergebnisse der erfindungsgemäßen Umsetzung von Methioninnitril mit der Stickstoffbase Guanidin als Katalysator und Aceton als Carbonylkatalysator (Beispiele 7 bis 13) mit den herkömmlichen Umsetzungen von Methioninnitril mit den Alkalibasen (in Form von NaOH) und Aceton/Cyclohexanon als Carbonylkatalysator zeigt (Vergleichs-Beispiele: 2, 3) zeigt, führt das erfindungsgemäße Guanidinsystem zu überraschenden Verbesserungen. So erzielt das erfindungsgemäße Verfahren bei gleich niedrigem Basenanteil von 0,10 Moleq und mit Aceton als Carbonylkatalysator sogar deutlich höhere Ausbeuten von ca. 99 % Methioninamidequivalenten (Beispiel 9) im Vergleich zur herkömmlichen Verfahrensweise mit nur 92,7 % (Vergleichs-Beispiel 2). Gleichzeitig wird in nur in minimalen Mengen von 0,2 Mol% das unerwünschte Rückspaltungsprodukt MMP (Beispiel 9) im Vergleich zu 3 Mol% beim NaOH-System gebildet, was einen außerordentlichen Vorteil darstellt.

Des Weiteren bevorzugt werden Verfahren der vorstehend bezeichneten Art, dadurch gekennzeichnet, dass bei der Umsetzung gemäß Schritt a. 1 bis 10 Moleq Ammoniak eingesetzt werden bezogen auf die molare Summe aus Methylmercaptopropionaldehyd und MMP-Cyanhydrin, so wie weiter oben angegeben, jedoch vorzugsweise 2 bis 8 Moleq Ammoniak und besonders bevorzugt 4 bis 7 Moleq Ammoniak. Insbesondere die Ammoniaküberschüsse gewährleisten, dass nur sehr wenig, nämlich ca. 1 Mol% des dimeren Iminodinitrilproduktes DN1 als Nebenprodukt entstehen. Die günstigsten Reaktionstemperaturen liegen bei 50 bis 100 °C. 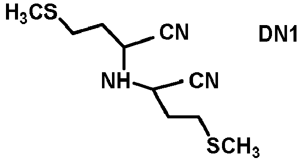 Des Weiteren bevorzugt werden Verfahren der vorstehend bezeichneten Art, dadurch gekennzeichnet, dass bei der Umsetzung gemäß Schritt a. wäßriger Ammoniak in einer Konzentration von 25 bis 80 Gew.-%, vorzugsweise von 30 bis 60 Gew.-% eingesetzt wird. In der Regel genügt es mit ca. 32 Gew.-% Ammoniak zu arbeiten, was den zusätzlichen Vorteil bietet, dass der eingesetzte Ammoniak dabei noch bei Normaldruck zu handhaben ist. Auch die höhere Ammoniakkonzentration trägt zu einer Verringerung der Iminodinitrilbildung bei.

Auch bevorzugt werden Verfahren der vorstehend bezeichneten Art, dadurch gekennzeichnet, dass die optionale Abtrennung von Ammoniak gemäß Schritt b. bei einem Druck von 0,01 bis 3 bara und einer Temperatur von 20 bis 70 °C, vorzugsweise von 0,1 bis 1 bara und einer Temperatur von 25 bis 60 °C erfolgt. Hierdurch werden die Volumenströme im hinteren Verfahrensabschnitt entlastet und der Reinigungsaufwand für den so isolierten recyclierbaren Ammoniak verringert. Dabei wird die noch heiße wässrig-ammoniakalische Lösung durch zumindest teilweise Entspannung von der Reaktionstemperatur von ca. 50 bis 100°C auf Temperaturen bis max. 40 bis 70 °C abgekühlt. Zwangsläufig wird dabei auch immer ein gewisser Anteil Wasser mit abgetrennt.

Dabei vorteilhaft wird die Abtrennung des Ketons und gemäß Schritt d. bei einem Druck von 0,001 bis 1 bara und einer Temperatur von 20 bis100 °C, vorzugsweise von 0,01 bis 0,9 bara und einer Temperatur von 25 bis 80 °C durchgeführt, weil so die maximale Ausbeute bei geringsten Nebenproduktanteilen und vergleichsweise kurzen Reaktionszeiten erhalten wird.
Besonders vorteilhaft ist auch eine Verfahrensweise, dadurch gekennzeichnet, dass das gemäß Schritt d. abgetrennte Keton zur Hydrolyse des Methioninnitrils in Schritt c. zurückgeführt werden, und so Verluste und Kosten minimiert werden. Dies kann unmittelbar oder wenn zweckmäßig nach einer gewissen Zwischenreinigung z.B. durch Destillation geschehen.

Im letzten Schritt bei der Hydrolyse des Methioninamids zum Methionin können sowohl saure als auch alkalische oder neutrale Katalysatoren verwendet werden.
Bei der Hydrolyse des Methioninamids zum Methionin gemäß Schritt e. kann als saurer Katalysator eine starke Mineralsäure, vorzugsweise Salzsäure, Schwefelsäure oder Phosphorsäure verwendet werden, welche allesamt sehr wirkungsvoll und relativ kostengünstig erhältlich sind. Allerdings führt die mineralsaure Hydrolyse primär zu Methionin-Mineralsäuresalzen, wodurch in diesem Fall zur Isolierung von freiem Methionin wiederum eine Neutralisation mit Base unter Salzanfall nötig wäre.

Bei Verwendung eines basischen Katalysator bei der Hydrolyse des Methioninamids gemäß Schritt e. wird vorzugsweise eine Alkali- oder Erdalkali- Base verwendet, insbesondere NaOH, KOH, Mg(OH)₂, Ca(OH)₂ oder Ba(OH)₂. Vorteilhaft dabei ist die schnelle und vollständige Hydrolyse bei moderaten Temperaturen. Nachteilig ist jedoch dabei, dass das Methionin zunächst als Alkali- oder Erdalkalisalz anfällt, aus dem erst wieder durch Neutralisation mit Säure das Methionin freigesetzt werden muss, was zwangsläufig wiederrum zu einem Salzanfall führt. Vorteilhaft ist die basische Hydrolyse dagegen, wenn sowieso ein Alkali- oder Erdalkalimethioninat als Produktform gewünscht ist, da diese dann auf direktem Weg als Endprodukt erhältlich ist.

Die besonders bevorzugte Verfahrensweise ist daher die Hydrolyse des Methioninamids zum Methionin mit Hilfe von neutralen Katalysatoren, weil hier der Salzanfall komplett vermieden werden kann.

Als neutraler Katalysator bei der Hydrolyse des Methioninamids gemäß Schritt e. können wiederum Titandioxide verwendet werden, welche vorteilhafterweise kein nennenswertes Leaching-Verhalten aufweisen, jedoch in effektiver Weise den abschließenden Hydrolyseschritt katalysieren. Durch derartige Verfahren werden in vorteilhafter Weise die Bildung von Salzabfallstoffen vermieden und es wird ein insgesamt salzarmes Verfahren zur Herstellung von Methionin bereitgestellt, was von erheblichem Vorteil ist, wie eingangs dargestellt. Das dabei primär entstehende Ammoniummethioninat kann anschließend leicht thermisch gespalten werden in Methionin, das dabei als Kristallisat anfällt und flüchtigen Ammoniak, der leicht abgetrennt und z.B. in die Aminonitrilsynthesestufe zurückgeführt werden kann.
Ebenso wurde in diesem Zusammenhang gefunden, dass unter den vielen Titanoxiden, die für den Hydrolyseschritt vom Methioninamid zum Methionin Ammoniumsalz getestet wurden, der kommerziell erhältliche Hombikat F01 (Sachtleben/Huntsman, pH-Wert = 6, spezifische Oberfläche = 350 m²/g, Titandioxidgehalt (geglüht) = 88 %, enthält 0,5% Schwefelsäure)) die besten Resultate mit quantitativem Umsatz/Ausbeute bei kurzer Reaktionsdauer ergibt. Diese sind auch auf kommerziellem Weg leicht verfügbar, würden aber auch nicht verloren, sondern nach ihrer Verwendung zurückgewonnen und recycliert.

Dabei vorteilhaft wird die Hydrolyse des Methioninamids gemäß Schritt e. bei Temperaturen von 70-180°C, vorzugsweise von 100-140°C durchgeführt. Dadurch wird insbesondere eine vollständige Umsetzung erreicht und damit ein qualitativ hochwertiges von Anfang an sehr reines Endprodukt.

Dabei bevorzugt wird ein Verfahren, dadurch gekennzeichnet, dass vor der Hydrolyse des Methioninamids zum Methionin mit einem neutralen Katalysator gemäß Schritt e. der vorhandene basische Katalysator mit Säure, vorzugsweise Schwefelsäure, Phosphorsäure, Salzsäure, Ameisensäure oder Kohlensäure zum entsprechenden Salz neutralisiert und ggf. abgetrennt wird. Bei diesem Salz handelt es sich je nach verwendeter Säure um Guanidiniumchlorid, Guanidiniumcarbonat, Guanidiniumhydrogencarbonat oder Guanidiniumsulfat, Guanidiniumhydrogensulfat, Guanidiniumphosphat, Guanidiniumhydrogenphosphat oder Guanidiniumdihydrogenphosphat.

Insbesondere das Guanidiniumdihydrogenphosphat, welches relativ schwer löslich ist in wässrigen Systemen (ca. 0,1 g/1 ml entsprechend 10 g pro 100 g Wasser), oder auch das Guanidiniumhydrogenphosphat (Wasserlöslichkeit gemäß US 2,469,338: 15,5 g pro 100 g Wasser bei 20 °C)können durch geschickte Wahl der Temperaturen und Konzentrationsbedingungen ohne größeren Aufwand weitgehend entfernt werden. Dieses kann direkt als hoch stickstoffhaltiger Phosphatdünger bzw. zur Herstellung eines solchen verwendet werden.

Eine weitere Möglichkeit besteht in der Abtrennung des Guanidins auf der Stufe des Methionins nach erfolgter Hydrolyse von Methioninamid. Dazu wird das Guanidin entweder noch vor der Hydrolyse oder danach in ein gut wasserlösliches Guanidiniumsalz durch Zugabe einer der oben genannten Säuren wie zum Beispiel von Kohlensäure in Form von CO₂ überführt, wobei in diesem Fall entsprechend Guanidiniumcarbonat entsteht. Die Zugabe der Säure nach der Verseifung hat den Vorteil, dass der noch vorhandene Anteil an basischem Guanidin während der Verseifung von Methioninamid zum Methionin als zusätzlicher Verseifungskatalysator neben dem z.B. hauptsächlich verwendeten neutralen Verseifungskatalysator TiO₂ unterstützend wirken kann.

Die Abtrennung kann vorteilhaft durch Auskristallisation des relativ schwer wasserlöslichen Methionins erfolgen, wobei die leichter als Methionin wasserlöslichen Salze des Guanidins in der Mutterlauge verbleiben, die entweder aufgearbeitet werden kann, so dass daraus wiederum durch Kristallisation das betreffende Salz erhalten werden kann oder direkt als Flüssigprodukt oder als Festprodukt nach Eindampfung, insbesondere nach Sprühgranulation an die Düngemittelindustrie oder auch an Anwender abgegeben werden kann zur Verwendung als Flüssig- oder Feststoffdünger oder zu dessen Herstellung. Da die so ausgeschleusten Guanidiniumsalze als hochstickstoffhaltige organische Stickstoffdünger begehrt sind, ist es hier erfindungsgemäß gelungen, noch ein weiteres wertvolles Produkt bereitzustellen. Da auf der anderen Seite das Verfahren so durchgeführt werden kann, dass nur 0,08 bis 0,12 Moleq der entsprechenden Salze bezogen auf das Methioninprodukt anfallen, halten sich bedingt durch die relativ kleinen Mengen die typischen Nachteile einer Koppelproduktion in Grenzen und es ist auf diese Weise auch gelungen, ein sehr ökonomisches wie auch ökologisch vertretbares Verfahren zur Methioninherstellung bereitzustellen.

Im Falle der bevorzugten Verwendung von CO₂ zur Neutralisation unter Bildung von Guanidiniumcarbonat ist es aber auch möglich, sowohl das Guanidin als auch das CO₂ zurückzugewinnen und im Verfahren wieder zu verwenden.

Es ist beispielsweise bekannt, dass Guanidiniumcarbonat bei Temperaturen von 80 bis 120 °C in Guanidin und CO₂ aufgespalten werden kann (Angew. Chem. Int. Ed. 2017, 2017, 56, 1042-1045). Daher wird nach der Hydrolyse des Methioninamids zum Methionin-Ammoniumsalz zweckmäßigerweise zunächst durch Einengen des Methionin-Ammoniumsalz-haltigen Hydrolysats das schwerlösliche Methionin ausgefällt und durch Kristallisation abgetrennt und filtriert sowie freigesetzter Ammoniak dabei ausgetrieben. Der Ammoniak kann wiedergewonnen werden und z,B. in der Aminonitrilbildungsstufe wiederverwendet werden. Die dabei erhaltene Mutterlauge enthält Guanidiniumcarbonat neben kleinen Anteilen an Methionin. Das Guanidiniumcarbonat kann durch einfache thermische Nachbehandlung der Guanidiniumcarbonat-haltigen Mutterlauge in eine stark basische Guanidin-haltige Lauge und flüchtiges CO₂ gespalten werden. Beide Ströme können wiedergewonnen werden.

Zur Erfindung gehört folglich auch ein Verfahren, das dadurch gekennzeichnet ist, dass die bei Verwendung von CO₂ als Säure in Schritt g. erhaltene Guanidiniumcarbonat enthaltende und in Schritt h. abgetrennte Mutterlauge eingedampft wird, vorzugsweise bei Temperaturen von 80 bis 120°C, zur thermischen Spaltung des Guanidiniumcarbonates in CO₂, welches ausgetrieben wird, und eine Guanidin enthaltene basische Mutterlauge.

Die Guanidin enhaltende basische Mutterlauge kann erfindungsgemäß als basischer Katalysator in Schritt c,, der Methioninamidstufe, wiederverwendet werden. Kleine Reste von Methionin in der Mutterlauge sind hier keinesfalls von Nachteil, sondern werden auf diese Weise automatisch ins Verfahren zurückgeführt, was einen weiteren großen Vorteil darstellt. Auf der anderen Seite kann das zurückgewonnene CO₂ zur Abtrennung des Guanidins ebenfalls wiederverwendet werden, wodurch der Kreis geschlossen wird.

Somit ist es gelungen ein technisches Herstellungsverfahren für Methionin bereitzustellen, das ausgehend von den verfügbaren Ausgangsstoffen MMP, Blausäure und Ammoniak über die milde carbonylkatalysierte Hydrolyse zum Methioninamid bei deutlich niedrigeren Temperaturen als beim derzeitigen Standardverfahren über die Hydantoin-Hydrolyse in hohen Ausbeuten und bei einfacherer Verfahrensführung zum Methionin führt.

Da das Prinzip des erfindungsgemäßen Verfahrens zur Herstellung von Methioninamid bzw. Methionin auch auf andere α-Aminosäuren anwendbar ist, ist auch der Gegenstand der Erfindung generell ein Verfahren zur Herstellung eines α-Aminosäureamids sowie der entsprechenden α-Aminosäure durch Hydrolyse des entsprechenden α-Aminosäurenitrils in Gegenwart eines Ketons und von Guanidin als Katalysator.

Gegenstand der Erfindung ist damit auch ein Verfahren zur Herstellung eines α-Aminosäureamids durch Hydrolyse des entsprechenden α-Aminosäurenitrils in Gegenwart eines Ketons und von Guanidin als Katalysator, welches die eingangs am Beispiel des Verfahrens zum Methioninamid/Methionin beschriebenen Vorteile aufweist. Vorzugsweise handelt es sich bei der α-Aminosäure dabei um Alanin, Homoserin, Serin, Threonin, Valin, Leucin, Isoleucin, Lysin, Methionin, Tryptophan, Histidin, Phenylalanin, Glycin oder Tyrosin.

Ganz analog werden auch hier erfindungsgemäß bei der Hydrolyse des α-Aminosäurenitrils mit der Base Guanidin entsprechend
als Keton vorzugsweise Aceton, Methyl-ethylketon, Diethylketon, Methyl-propylketon, Methyl-isopropylketon oder Ethyl-propylketon, Cyclopentanon, Cyclohexanon oder 4-Piperidon, besonders bevorzugt Aceton, Methyl-ethylketon oder 4-Piperidon eingesetzt.

Auf diese Weise wurde erfindungsgemäß ein Verfahren bereitgestellt zur Herstellung einer α-Aminosäure umfassend einen ersten Schritt a) der Hydrolyse des entsprechenden α-Aminosäurenitrils zum entsprechenden α-Aminosäureamid in Gegenwart eines Ketons und von Guanidin als Katalysator und einen zweiten Schritt b) der Weiterhydrolyse des α-Aminosäureamids zur entsprechenden α-Aminosäure. Dabei sind die vorstehend anhand von Methionin dargestellten Verfahren anwendbar.
Grundlegender Gegenstand der vorliegenden Erfindung ist somit generell die Verwendung von Guanidin als basischer Katalysator zur Carbonyl-katalysierten Hydrolyse von α-Aminosäurenitrilen zu den entsprechenden α-Aminosäureamiden, vorzugsweise zur Hydrolyse von Methioninnitril zu Methioninamid.

### Beispiele:

### Analytische Methoden

Die chromatographischen Untersuchungen (MMP-Cyanhydrin, MMP, Methionin, Methioninamid, Methioninnitril, IM1, IM2) wurden mittels HPLC der Firma JASCO an einer RP-18 Säule (250 x 4,6 mm; 5 µm) mit anschließender UV Detektion bei 210 nm durchgeführt. Als Laufmittel diente ein phosphorsaures Acetonitril-Wasser-Gemisch (3,3 g H₃PO₄, 6,8 g Acetonitril, 89,9 g H₂O). Bei einem Fluss von 1 mL/min wurden 10 µL der jeweiligen Probelösung (50 mg Probe in 25 mL H₂O) injiziert. Die Kalibrierung erfolgte im Vorfeld durch die Injektion geeigneter Kalibrierlösungen, die Auswertung erfolgte durch Peakflächenvergleich mittels der externen Standardmethode. Die Durchführung der Standardmethode ist dem Fachmann bekannt.

### Beispiel 1: Synthese von Methioninnitril ausgehend von MMP-Cyanhydrin

64,0 g MMP-Cyanhydrin (90% in Wasser, 0,439 mol, 1 Moleq) wurde im 1 L-Reaktor bei 40 °C vorgelegt und unter Rühren mit 168,0 g Ammoniak (32 Gew.% in Wasser, 7 Moleq, 3,107 mol) versetzt (MMP-Cyanhydrin-Konzentration 25 Gew.%). Die beigefarbene und trübe, aber gut rührbare Emulsion wurde auf 50 °C temperiert und 30 min gerührt (ein Überschreiten von 55 °C durch die Exothermie der Reaktion wurde dabei vermieden). Die erhaltene klare, hellgelbe Lösung zeigte mittels HPLC-Analytik eine 100%-ige Umsetzung des MMP-Cyanhydrins bei 98,8%-iger Selektivität zu Methioninnitril-Äquivalenten auf (Methioninnitril (0,422 mol) und Methioninamid (0,011 mol). In Spuren ist eine Bildung des Iminodinitrils 2,2'-Bis-(2-methylmercaptoethyl)-iminodiacetonitril (DN1, <0,1%;) zu beobachten.

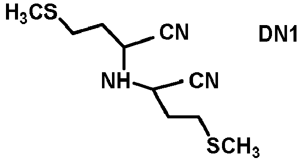

### Beispiel 2 (Vergleich): Hydrolyse von MMP-Aminonitril mit NaOH, Ammoniak und Aceton

Wie in Beispiel 1 beschrieben wurde eine Reaktionslösung enthaltend 54,9 g Methioninnitril (0,422 mol), 1,6 g Methioninamid (0,011 mol; Methioninnitril und Methioninamid bilden zusammen 1 Moleq), 45,4 g Ammoniak (2,67 mol, 6 Moleq) und 120 g Wasser hergestellt, auf 35 °C abgekühlt und dann nacheinander mit 43 g Wasser, 25 g Aceton (1 Moleq, 0,43 mol) sowie mit 10 mol% einer NaOH-Lösung (10 Gew.% in Wasser; 0,043 mol) versetzt (Methioninnitrilkonzentration ca. 17 Gew.%).

Das Reaktionsgemisch wurde 1,5 Stunden bei 35 °C gerührt. Die erhaltene klare, gelbe Lösung wurde mittels HPLC analysiert und der Umsatz des Methioninnitrils sowie Selektivität zu Methioninamid-Äquivalenten (Methioninamid, Natriummethioninat sowie IM2) bestimmt. Dabei wurde eine 100%-ige Umsetzung des Methioninnitrils bei 92,7%- iger Selektivität bzw. Ausbeute an Methioninamid-Äquivalenten gefunden. Der Anteil an IM2, welches bei Temperaturerhöhung Aceton freisetzt und sich praktisch wie Methioninamid verhält, betrug 2,5 Mol%. In Spuren war eine weitere Imidazolidinon-Komponente (IM1) aus der Reaktion mit MMP sowie das beschriebene Iminodinitril DN1 samt Folgeprodukte (<0,1%), bei denen die beiden Nitril-Funktionen vollständig der zum Teil zu den entsprechenden Amiden oder Carbonsäuren umgesetzt wurden, zu beobachten. Daneben wurde die Bildungsrate der Rückspaltungsprodukte MMP und MMP-Cyanhydrin zu 3 bzw. 0 Mol% bestimmt.

Über die beiden Stufen ergab sich somit ausgehend von 0,439 mol MMP- Cyanhydrin eine Ausbeute an Methioninamid -Äquivalenten von 92,7% d.Th. (0,407 mol).

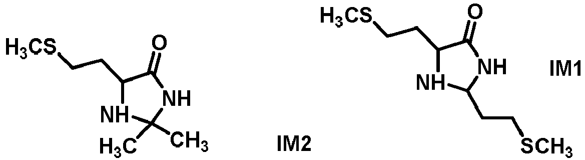

### Beispiel 3 (Vergleich): Hydrolyse von MMP-Aminonitril mit NaOH, Ammoniak und Cyclohexanon

Methioninnitril (1 Moleq) wurde als 29 gewichtsproz. wässrig-ammoniakalische Lösung (enthaltend 0,3 Moleq Ammoniak und 17,5 Moleq Wasser) bei einer Starttemperatur von 30 °C vorgelegt und verdünnte NaOH (0,2 Moleq) sowie Cyclohexanon (0,2 Moleq) zugegeben und die Mischung anschließend bei ca .35 °C 0,5 h lang gerührt. Es wurde eine Ausbeute von 78,6% Methioninamid sowie von 2,3% Methioninnatriumsalz (80,9 % Methioninamid -Äquivalenten) via HPLC detektiert.

### Beispiel 4: Hydrolyse von Methionin-Amid mit TiO₂ zu Methionin und Ammoniak

In einem Rundkolben mit Rückflusskühler wurden Methioninamid (30 mmol, 9.0 g einer 50% wässrige Lösung), Hombikat F01 (1.0 g Pulver) und Wasser (80 mL) eine Stunde bei 85 °C Badtemperatur gekocht. Es konnte eine Ausbeute von 4.43 g (>99%) Methionin via HPLC detektiert werden.

### Beispiel 5: Freisetzung von Guanidin aus dem Guanidiniumchlorid:

Zur Freisetzung von Guanidin aus Guanidiniumchlorid wurde Ambersep 900 OH als basischer Ionenaustauscher verwendet. Ambersep 900 OH ist ein stark basischer Anionenaustauscher mit makroretikülärer Struktur. Er besteht aus einer Styren-Divinylbenzen-Matrix und ist beladen mit quartären Ammoniumverbindungen.

Zur Freisetzung wurde eine Säule mit 40 mL Ambersep 900 OH gefüllt. Es wurden 100ml NaOH 4w% langsam über den Tropftrichter zugegeben. Anschließend wurde so lange mit VE-Wasser nachgewaschen, bis der pH-Wert der unter der Säule austropfenden Lösung zwischen pH 6 und pH 7 lag. Danach wurde eine Lösung bestehend aus 5,0 g Guanidiniumchlorid und 15 g Wasser langsam zugetropft und mit 150 ml Wasser nachgespült. Die aufgefangene Guanidin-Lösung wurde am Rotationsverdampfer bei 30 mbar und 45°C auf eine Guanidinkonzentration von 20 Gew.-% eingestellt und umgehend für weitere Reaktionen eingesetzt. Die pH-Wert Messung erfolgte mit einem pH-Meter mit Einstabmesskette.

### Beispiel 6 - 13: Hydrolyse von Methioninnitril mit Guanidin und Aceton als Carbonylkatalysator bei verschiedenen Temperaturen bzw. Konzentrationen

Methioninnitril (1 Moleq) wurde als 14 gewichtsproz. wässrig-ammoniakalische Lösung (enthaltend 6 Moleq Ammoniak) vorgelegt und Aceton (0-1,2 Moleq) sowie eine 20 Gew.%-ige wässrige Guanidin-Lösung (0,01-1,2 Moleq, wie in Tab.1 angegeben), hergestellt nach Beispiel 5, zugegeben und die Mischung anschließend bei einer Temperatur von 35 °C 2,5 h lang gerührt und analog wie bei o.g. Beispielen der Umsatz, die Selektivität und die Ausbeute an den unmittelbaren Methioninvorstufen (Methioninamid, IM2) und anteilig bereits gebildetem Methionin (angegeben als Gesamtwert Ausbeute) sowie die Bildungsrate der Rückspaltungsprodukte MMP und MMP-Cyanhydrin bestimmt (vgl. Tab.1).

**Tabelle 1: Hydrolyse von Methioninnitril mit verschiedenen Anteilen Guanidin und und Aceton als Carbonylkatalysator**

| **Beispiel** | **Aceton [Moleq]** | **Guanidin [Moleq]** | **Ausbeute [%]** | **Umsatz [%]** | **Selektivität [%]** | **MMP-Bildung [Mol%]** | **MMP-CH-Bildung [Mol%]** |
|---|---|---|---|---|---|---|---|
| 6 (Vergleich) | 0 | 1,2 | 48 | 52 | 92 | <4% | <1 |
| 7 | 1,2 | 1,2 | 98 | 100 | 98 | <0,5 | 0 |
| 8 | 1,2 | 0,5 | 100 | 100 | 100 | <0,2 | 0 |
| 9 | 1,2 | 0,1 | 99 | 100 | 99 | <0,2 | 0 |
| 10 | 1,2 | 0,08 | 85 | 95 | 90 | <0,6 | 0 |
| 11 | 1,2 | 0,05 | 84 | 92 | 91 | <1 | 0 |
| 12 | 1,2 | 0,03 | 74 | 85 | 87 | <2 | 0 |
| 13 | 1,2 | 0,01 | 64 | 75 | 85 | <3 | <0,1 |

Die Ergebnisse in Tabelle 1 zeigen in unvorhersehbarer Weise, dass mit der starken organischen Stickstoffbase Guanidin unter Carbonylkatalyse bei moderaten Bedingungen Methioninnitril in quantitativer Ausbeute zu Methioninamid hydrolysiert werden kann. Bereits bei Guanidin-Anteilen von nur 0,03 Moleq wurden bei der hier gewählten Temperatur/Zeit-Kombination relativ hohe Ausbeuten an den gewünschten Methioninvorstufen (74%) erhalten. Mit nur 0,05 Moleq Guanidin bei 1,2 Moleq Aceton wurden bereits 92 % Umsatz bei 91 % Selektivität erzeugt, und es waren dabei trotzdem nur <1 % MMP nachweisbar, was bedeutet, dass die unerwünschte Rückreaktion des Aminonitrils zum Aldehyd und Blausäure in nur geringem Ausmaß stattfindet. Das ist sehr vorteilhaft, da das MMP die Aufarbeitung bzw. Endproduktreinigung in deutlicher Weise stören würde. Mit 0,1 Moleq Guanidin bei 1,2 Moleq Aceton wurden dagegen schon 100 % Umsatz bei 99 % Selektivität erzeugt, was einer praktisch quantitativen Ausbeute entspricht. Dabei wurden sogar nur noch < 0,2 Mol% an unerwünschtem MMP gebildet, was einen sehr guten Wert darstellt.

## Patentansprüche

1. Verfahren zur Herstellung von Methioninamid durch Hydrolyse von Methioninnitril in Gegenwart eines Ketons und von Guanidin als Katalysator.

2. Verfahren zur Herstellung von Methioninamid **gekennzeichnet durch** folgende Schritte:
a. Herstellung von Methioninnitril durch Umsetzung von Methylmercaptopropionaldehyd (MMP) mit Blausäure und Ammoniak und/oder durch Umsetzung des entsprechenden MMP-Cyanhydrins (MMP-CH) mit Ammoniak zu einem Methioninnitril enthaltendem Reaktionsgemisch,
b. optional wenigstens teilweise Abtrennung von restlichem Ammoniak aus dem Reaktionsgemisch aus a.,
c. Hydrolyse des Methioninnitrils zum Methioninamid im Reaktionsgemisch aus a. oder b. in Gegenwart eines Ketons und von Guanidin als Katalysator und
d. optional Abtrennung des Ketons und/oder des Guanidins aus dem Reaktionsgemisch aus c.

3. Verfahren zur Herstellung von Methionin **gekennzeichnet durch** folgende Schritte:
a. Herstellung von Methioninnitril durch Umsetzung von Methylmercaptopropionaldehyd (MMP) mit Blausäure und Ammoniak und/oder durch Umsetzung des entsprechenden MMP-Cyanhydrins (MMP-CH) mit Ammoniak zu einem Methioninnitril enthaltenden Reaktionsgemisch,
b. optional wenigstens teilweise Abtrennung von restlichem Ammoniak aus dem Reaktionsgemisch aus a.,
c. Hydrolyse von Methioninnitril zum Methioninamid im Reaktionsgemisch aus a. oder b. in Gegenwart eines Ketons und von Guanidin als Katalysator,
d. optional wenigstens teilweise Abtrennung des Ketons aus dem Reaktionsgemisch aus c.,
e. anschließender Hydrolyse des Methioninamids aus c. oder d. in Gegenwart eines basischen oder neutralen Katalysators,
f. Einengen des aus e. erhaltenen Hydrolysats zur Aufkonzentrierung und zu Entfernung von Ammoniak und restlichem Keton,
g. Neutralisation des aus f. erhaltenen aufkonzentrierten Hydrolysats bis zu einem pH-Wert von 5 bis 9 durch Zugabe entsprechender Mengen an Säure, vorzugsweise CO₂, zur Ausfällung von Methionin und gleichzeitiger Erzeugung einer Mutterlauge, die das zur zugegebenen Säure korrespondierende Guanidiniumsalz, vorzugsweise Guanidiniumcarbonat, enthält, und
h. Abtrennung des Methionins aus der Mutterlauge.

4. Verfahren gemäß Anspruch 1, 2 oder 3, **dadurch gekennzeichnet, dass** bei der Hydrolyse des Methioninnitrils 0,01 bis 1,2, vorzugsweise 0,05 bis 1,0, besonders bevorzugt 0,08 bis 0,5 Moleq Guanidin eingesetzt werden.

5. Verfahren gemäß einem der Ansprüche 1 bis 4, **dadurch gekennzeichnet, dass** bei der Hydrolyse des Methioninnitrils als Keton Aceton, Methyl-ethylketon, Diethylketon, Methyl-propylketon, Methyl-isopropylketon, Ethyl-propylketon, Cyclopentanon, Cyclohexanon oder 4-Piperidon, vorzugsweise Aceton, Methyl-ethylketon oder 4-Piperidon eingesetzt werden.

6. Verfahren gemäß einem der Ansprüche 1 bis 5, **dadurch gekennzeichnet, dass** die Hydrolyse des Methioninnitrils bezogen auf das Nitril in Gegenwart von 15 bis 50 Moleq H₂O, 0,1 bis 1,5 Moleq Keton und 0 bis 9 Moleq NH₃ sowie bei einer Temperatur von 10 bis 50°C und in einer Reaktionszeit von 10 bis 180 Minuten durchgeführt wird.

7. Verfahren gemäß Anspruch 2 oder 3, **dadurch gekennzeichnet, dass** bei der Umsetzung gemäß Schritt a. 1 bis 10 Moleq Ammoniak, vorzugsweise 2 bis8 Moleq Ammoniak und besonders bevorzugt 4 bis 7 Moleq Ammoniak eingesetzt werden bezogen auf die molare Summe aus Methylmercaptopropionaldehyd und MMP-Cyanhydrin.

8. Verfahren gemäß Anspruch 2 oder 3, **dadurch gekennzeichnet, dass** bei der Umsetzung gemäß Schritt a. wäßriger Ammoniak in einer Konzentration von 25 bis 80 Gew.-%, vorzugsweise von 30 bis 60 Gew.-%, eingesetzt wird.

9. Verfahren gemäß Anspruch 2 oder 3, **dadurch gekennzeichnet, dass** die Abtrennung von restlichem Ammoniak gemäß Schritt b. bei einem Druck von 0,01 bis 3 bara und einer Temperatur von 20 bis 70 °C, vorzugsweise von 0,1 bis 1 bara und einer Temperatur von 25 bis 60 °C erfolgt.

10. Verfahren gemäß Anspruch 2 oder 3, **dadurch gekennzeichnet, dass** die Abtrennung des Ketons gemäß Schritt d. bei einem Druck von 0,001 bis 1 bara und einer Temperatur von 20 bis 100 °C, vorzugsweise von 0,01 bis 0,9 bar und einer Temperatur von 25 bis 80 °C erfolgt.

11. Verfahren nach den Ansprüchen 2, 3 oder 10, **dadurch gekennzeichnet, dass** das gemäß Schritt d. abgetrennte Keton zur Hydrolyse des Methioninnitrils in Schritt c. zurückgeführt werden.

12. Verfahren gemäß Anspruch 3, **dadurch gekennzeichnet, dass** bei der Hydrolyse des Methioninamids gemäß Schritt e. als saurer Katalysator eine starke Mineralsäure, vorzugsweise Salzsäure, Schwefelsäure oder Phosphorsäure verwendet wird.

13. Verfahren gemäß Anspruch 3, **dadurch gekennzeichnet, dass** bei der Hydrolyse des Methioninamids gemäß Schritt e. als basischer Katalysator ein Alkali- oder Erdalkali- Base, vorzugsweise NaOH, KOH, Mg(OH)₂, Ca(OH)₂ oder Ba(OH)₂, verwendet wird.

14. Verfahren gemäß Anspruch 3, **dadurch gekennzeichnet, dass** bei der Hydrolyse des Methioninamids gemäß Schritt e. als neutraler Katalysator Titandioxid, verwendet wird.

15. Verfahren nach einem oder mehreren der Ansprüche 3 bis 14, **dadurch gekennzeichnet, dass** die Hydrolyse des Methioninamids gemäß Schritt e. bei Temperaturen von 70-180°C, vorzugsweise von 100-140°C durchgeführt wird.

16. Verfahren nach einem oder mehreren der Ansprüche 3 bis 15, **dadurch gekennzeichnet, dass** die bei Verwendung von CO₂ als Säure in Schritt g. erhaltene, Guanidiniumcarbonat enthaltende und in Schritt h. abgetrennte Mutterlauge eingedampft wird, vorzugsweise bei Temperaturen von 80-120°C, zur thermischen Spaltung des Guanidiniumcarbonates in CO₂, welches ausgetrieben wird, und eine Guanidin enthaltene basische Mutterlauge.

17. Verfahren nach Anspruch 16, **dadurch gekennzeichnet, dass** die Guanidin enthaltende basische Mutterlauge als Guanidin-Katalysator in Schritt c, wiederverwendet wird.

18. Verfahren zur Herstellung eines α-Aminosäureamids durch Hydrolyse des entsprechenden α-Aminosäurenitrils in Gegenwart eines Ketons und von Guanidin als Katalysator.

19. Verfahren gemäß Anspruch 18, **dadurch gekennzeichnet, dass** es sich bei dem α-Aminosäureamid um das jeweilige Amid der α-Aminosäure Alanin, Serin, Homoserin, Threonin, Valin, Leucin, Isoleucin, Lysin, Methionin Tryptophan, Histidin, Phenylalanin, Glycin oder Tyrosin handelt.

20. Verfahren gemäß einem der Ansprüche 18 oder 19, **dadurch gekennzeichnet, dass** bei der Hydrolyse des α-Aminosäurenitrils als Keton Aceton, Methyl-ethylketon, Diethylketon, Methyl-propylketon, Methyl-isopropylketon, Ethyl-propylketon, Cyclopentanon, Cyclohexanon oder 4-Piperidon, vorzugsweise Aceton, Methyl-ethylketon oder 4-Piperidon eingesetzt werden.

21. Verfahren zur Herstellung einer α-Aminosäure umfassend
einen ersten Schritt a) der Hydrolyse des entsprechenden α-Aminosäurenitrils zum entsprechenden α-Aminosäureamid gemäß einem der Ansprüche 16 bis 18 und
einen zweiten Schritt b) der Weiterhydrolyse des α-Aminosäureamids zur entsprechenden α-Aminosäure.

22. Verwendung von Guanidin als basischer Katalysator zur Carbonyl-katalysierten Hydrolyse von α-Aminosäurenitrilen zu den entsprechenden α-Aminosäureamiden, vorzugsweise zur Hydrolyse von Methioninnitril zu Methioninamid.
